# EUROPEAN PATENT APPLICATION

(11) **EP 4 074 257 A1**
(43) Date of publication of application: **19.10.2022**
(21) Application number: 20899438.4
(22) Date of filing: 01.12.2020
(51) Int. Cl.: A61B 5/1455, G01N 21/3577, G01N 21/359

(54) **MEASUREMENT SYSTEM, MEASURING DEVICE, AND PROGRAM**

(30) Priority: 11.12.2019 JP 2019223365
(71) Applicant: Quantum Operation, Inc., Tokyo, 104-0052 (JP)
(72) Inventor: KATO Kazuma, Tokyo 104-0052 (JP); YAMAGISHI Jyunichi, Tokyo 104-0052 (JP)
(74) Representative: Plougmann Vingtoft a/s
(86) International application number: PCT/JP2020/044640
(87) International publication number: WO 2021/117546

(57) **Abstract**

This measurement system S comprises: an infrared emission unit 11 that emits first infrared light; an infrared reception unit 15 that receives second infrared light, which is light obtained as a result of the first infrared light being reflected on a portion of the body of a measurement subject U or being transmitted through a portion of the body of a measurement subject U; an infrared current generation unit 13 that applies a pulse current to the infrared emission unit 11; a duty control part 202 that, on the basis of the intensity of the second infrared light, changes the duty of the pulse current applied by the infrared current generation unit 13; and a glucose level determination device 2 that, on the basis of the intensity of the second infrared light, determines the blood glucose level of the measurement subject U.

## Description

### TECHNICAL FIELD

The present disclosure relates to a measurement system, a measurement apparatus, and a program for measuring a blood glucose level.

### BACKGROUND ART

A conventional method of measuring a blood glucose level using pulse light with a near-infrared wavelength is known. Patent Document 1 discloses a technique for measuring a glucose concentration in blood by irradiating a wrist with pulse light having a near-infrared wavelength and detecting reflected pulse light reflected by the wrist.

### PRIOR ART

### PATENT DOCUMENT

Patent Document 1: Japanese Unexamined Patent Application Publication No. 2014-147404

### SUMMARY OF INVENTION

### PROBLEMS TO BE SOLVED BY THE INVENTION

In order to improve the accuracy in measuring the glucose concentration in blood, it is desirable to increase an intensity of the pulse light having the near-infrared wavelength. However, if current flowing through a light source is excessively increased in order to increase the emission intensity, a temperature of the light source will rise and the wavelength of the pulse light will change. Further, when a measurement subject is warmed by heat generated by the light source, the wavelength of the light absorbed by the glucose in the measurement subject's blood will also change. Since a wavelength range of the light absorbed by the glucose is narrow, there is a problem that the accuracy of measuring the amount of glucose will decrease if the wavelength of the pulse light or the wavelength of the light absorbed by the glucose changes.

The present disclosure focuses on this point, and an object thereof is to improve the accuracy of measuring the amount of glucose in blood.

### MEANS FOR SOLVING THE PROBLEMS

A first aspect of the present disclosure provides a measurement system including an infrared light emitter that emits first infrared light, an infrared light receiver that receives second infrared light that is (i) light resulting from the first infrared light being reflected by a part of a body of a measurement subject or (ii) light resulting from the first infrared light being transmitted through a part of the body of the measurement subject, a current generator for infrared light that causes a pulse current to flow to the infrared light emitter, a duty controller that changes a duty of the pulse current caused to flow by the current generator for infrared light on the basis of an intensity of the second infrared light, and a glucose amount identification part that identifies a glucose amount in blood of the measurement subject on the basis of an intensity of the second infrared light.

The measurement further includes a red light emitter that emits first red light, a red light receiver that receives second red light that is (i) light resulting from the first red light being reflected by a part of the body of the measurement subject or (ii) light resulting from the first red light being transmitted through a part of the body of the measurement subject, and a current generator for red light that causes a pulse current to flow through the red light emitter, wherein the duty controller may change the duty of the pulse current caused to flow through the infrared light emitter by the current generator for infrared light such that the glucose amount identification part can measure the glucose amount on the basis of the relationship between the intensity of the second infrared light and the intensity of the second red light.

The duty controller may control the current generator for red light such that the current generator for red light (i) causes a current to flow to the red light emitter to cause the red light emitter to radiate the first red light while the infrared light emitter is not emitting the first infrared light and (ii) causes a current not to flow to the red light emitter while the infrared light emitter is emitting the first infrared light.

The duty controller may change the duty of the pulse current such that an average current flowing through the infrared light emitter is equal to or less than the maximum current value allowed for the infrared light emitter.

The duty controller may generate a pulse voltage of a duty corresponding to the duty of the pulse current, the current generator for infrared light may include a first infrared transistor that causes a current to flow to the infrared light emitter while the pulse voltage has a first polarity, and a second infrared transistor that connects a terminal connected to the first infrared transistor in the infrared light emitter to a ground while the pulse voltage has a second polarity different from the first polarity.

The glucose amount identification part may identify the intensity of the second infrared light by integrating an amount of the second infrared light received by the infrared light receiver while the current generator for infrared light is causing a pulse current to flow through the infrared light emitter.

The measurement system further includes a storage that stores a value of the duty at a time when measurement by the glucose amount identification part is finished, wherein the duty controller may determine the duty at a time when the pulse current starts to flow through the infrared light emitter, on the basis of the value of the duty stored in the storage.

A second aspect of the present disclosure provides a measurement apparatus including an infrared light emitter that emits first infrared light, an infrared light receiver that receives second infrared light that is (i) light resulting from the first infrared light being reflected by a part of a body of a measurement subject or (ii) light resulting from the first infrared light being transmitted through a part of the body of the measurement subject, a current generator for infrared light that causes a pulse current to flow through the infrared light emitter, a duty controller that changes a duty of the pulse current caused to flow by the current generator for infrared light on the basis of an intensity of the second infrared light, and a transmission part that transmits data indicating the intensity of the second infrared light to a glucose amount identification apparatus that identifies a glucose amount in blood of the measurement subject on the basis of the intensity of the second infrared light.

A third aspect of the present disclosure provides a program for causing a computer to execute the steps of generating a pulse voltage of a first duty to be inputted to a current generator for infrared light that drives an infrared light emitter that emits first infrared light, changing a duty of the pulse voltage from the first duty to a second duty on the basis of an intensity of second infrared light that is (i) light resulting from the first infrared light being reflected by a part of a body of a measurement subject or (ii) light resulting from the first infrared light being transmitted through a part of the body of the measurement subject, and transmitting data indicating the intensity of the second infrared light to a glucose amount identification apparatus for identifying a glucose amount in blood of the measurement subject on the basis of the intensity of the second infrared light.

### EFFECT OF THE INVENTION

According to the present disclosure, it is possible to improve the accuracy of measuring the amount of glucose in blood.

### BRIEF DESCRIPTION OF THE DRAWINGS

FIG. 1 shows an overview of a measurement system.
FIG. 2 shows a configuration of a measurement apparatus.
FIG. 3 shows circuit examples of a current generator for infrared light and a current generator for red light.
FIG. 4 is a flowchart that shows processing executed by a controller.

### DETAILED DESCRIPTION OF THE INVENTION

### [Overview of measurement system S]

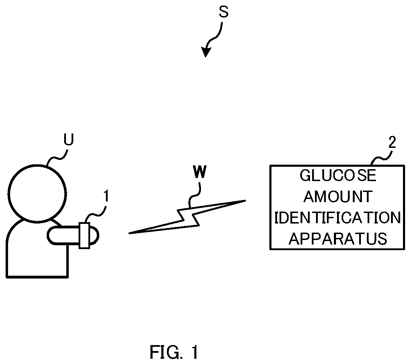

FIG. 1 shows an overview of a measurement system S. The measurement system S is a system for measuring an amount of glucose in a body of a measurement subject U. The measurement system S includes a measurement apparatus 1 and a glucose amount identification apparatus 2. The measurement apparatus 1 and the glucose amount identification apparatus 2 transmit and receive data by wireless communication or wired communication. In an example shown in FIG. 1, the measurement apparatus 1 and the glucose amount identification apparatus 2 transmit and receive data via a wireless channel W. The wireless channel W is Bluetooth (registered trademark) or Wi-Fi (registered trademark), for example.

The measurement apparatus 1 is an apparatus worn on a part of the body (for example, wrist) of the measurement subject U. The glucose amount identification apparatus 2 functions as a glucose amount identification part that measures the glucose amount in blood of the measurement subject U on the basis of the data received from the measurement apparatus 1, and is a computer or a smartphone, for example.

The measurement apparatus 1 irradiates the measurement subject U with pulsed light while the apparatus is attached to a part of the measurement subject U's body. Since some of the radiated light is absorbed by the glucose in a blood vessel, the intensity of reflected light reflected by the body or transmitted light transmitted through the body changes depending on the amount of glucose in the blood vessel. The measurement apparatus 1 detects the intensity of the reflected light or transmitted light, and transmits data indicating the detected intensity to the glucose amount identification apparatus 2 via the wireless channel W. The glucose amount identification apparatus 2 analyzes the glucose amount in the measurement subject U's blood on the basis of the intensity of the reflected light or transmitted light received from the measurement apparatus 1 to identify the glucose amount.

As described above, if current flowing through a light source is excessively increased in order to increase the light emission intensity, a temperature of the light source will rise and a wavelength of pulse light will change. Further, when the measurement subject U is warmed by heat generated by the light source, the wavelength of the light absorbed by the glucose in the measurement subject U's blood will also change. If the wavelength of the pulse light or the wavelength of the light absorbed by glucose changes, the accuracy of detecting the amount of glucose will decrease.

In order to solve this problem, the measurement apparatus 1 controls a duty of the pulse light to be radiated on the basis of the intensity of the reflected light or transmitted light, such that an increase in the temperature of the light source is suppressed while increasing the emission intensity. As a result, a change in the wavelength of the pulse light from the light source and a change in the wavelength of the light absorbed by the glucose in the measurement subject U's blood are suppressed, thus improving the accuracy of measuring the glucose amount. It should be noted that the duty is a ratio of a time during which the light is emitted to a time during which the measurement apparatus 1 is operating.

### [Configuration of measurement apparatus 1]

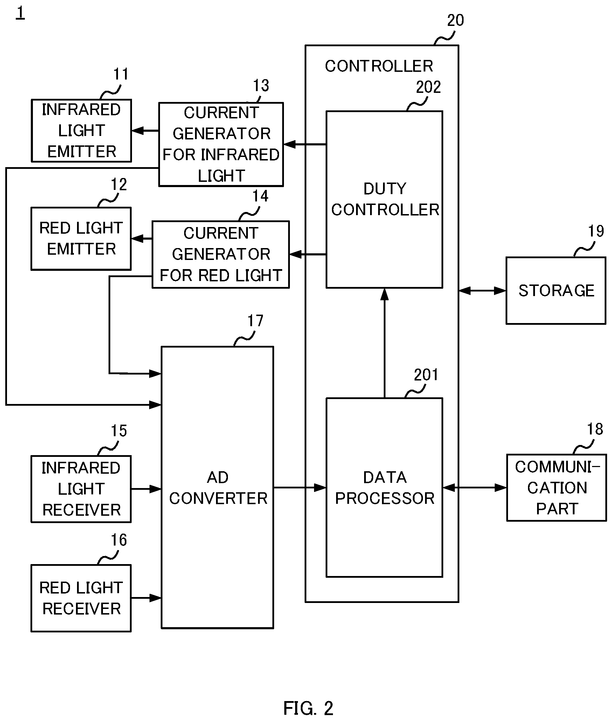

FIG. 2 shows a configuration of a measurement apparatus 1. The measurement apparatus 1 includes an infrared light emitter 11, a red light emitter 12, a current generator for infrared light 13, a current generator for red light 14, an infrared light receiver 15, a red light receiver 16, an AD converter 17, a communication part 18, a storage 19, and a controller 20. The controller 20 includes a data processor 201 and a duty controller 202.

The infrared light emitter 11 includes an optical element that emits infrared light. The wavelength of the infrared light emitted by the infrared light emitter 11 is between 800 nm and 2500 nm. In this disclosure, the infrared light emitted by the infrared light emitter 11 is referred to as first infrared light.

The red light emitter 12 includes an optical element that emits red light. The wavelength of the red light emitted by the red light emitter 12 is between 600 nm and 800 nm. In this disclosure, the red light emitted from the red light emitter 12 is referred to as first red light. It should be noted that the measurement apparatus 1 can identify the glucose amount by measuring the intensity of the reflected light or transmitted light of the first infrared light, but the measurement apparatus 1 uses the first red light together with the first infrared light, as an example, in order to prevent a decrease in measurement accuracy due to changes in the reflected light or transmitted light of the first infrared light caused by changes in body conditions (for example, body temperature or pulsation).

The current generator for infrared light 13 causes the infrared light emitter 11 to emit the light by causing a pulse current to flow through the infrared light emitter 11. As will be described in detail later, the current generator for infrared light 13 includes a circuit for causing the pulse current to flow through the infrared light emitter 11 on the basis of a pulse voltage inputted from the duty controller 202. In addition, the current generator for infrared light 13 inputs a signal indicating magnitude of the current flowing through the infrared light emitter 11 to the AD converter 17 such that an overcurrent in the infrared light emitter 11 can be detected.

The current generator for red light 14 causes the pulse current to flow to the red light emitter 12, to cause the red light emitter 12 to radiate the first red light. The current generator for red light 14 includes a circuit for causing the pulse current to flow through the red light emitter 12 on the basis of the pulse voltage inputted from the duty controller 202. The current generator for red light 14 causes the current to flow to the red light emitter 12 while the infrared light emitter 11 is not emitting the first infrared light, and does not cause the current to flow to the red light emitter 12 while the infrared light emitter 11 is emitting the first infrared light. Further, the current generator for red light 14 inputs a signal indicating the magnitude of the current flowing through the red light emitter 12 to the AD converter 17 such that the overcurrent in the red light emitter 12 can be detected.

The infrared light receiver 15 receives the first infrared light reflected by the part of measurement subject U's body or the second infrared light transmitted through the part of the measurement subject U's body. The second infrared light is infrared light having substantially the same frequency as the first infrared light, but is weaker than the first infrared light because some of the first infrared light is absorbed by the glucose or the like in the blood. The infrared light receiver 15 inputs a signal indicating the intensity of the received second infrared light to the AD converter 17.

The red light receiver 16 receives the first red light reflected by the part of the measurement subject U's body or the second red light transmitted through the part of the measurement subject U's body. The red light receiver 16 inputs a signal indicating the intensity of the received second red light to the AD converter 17.

The AD converter 17 converts the inputted analog signal into digital data. Specifically, the AD converter 17 converts, into digital data, each of the following: (i) an analog signal that varies in accordance with the current flowing through the infrared light emitter 11 inputted from the current generator for infrared light 13, (ii) an analog signal that varies in accordance with the current flowing through the red light emitter 12 inputted from the current generator for red light 14, (iii) an analog signal that indicates the intensity of the second infrared light inputted from the infrared light receiver 15, and (iv) an analog signal that indicates the intensity of the second red light inputted from the red light receiver 16. The AD converter 17 inputs the converted digital data to the data processor 201. The AD converter 17 inputs the digital data to the data processor 201 through Serial Peripheral Interface (SPI) communication, for example.

The communication part 18 includes a communication interface for transmitting and receiving data to and from the glucose amount identification apparatus 2. The communication part 18 includes a communication controller for Bluetooth, for example. The communication part 18 transmits the data inputted from the data processor 201 to the glucose amount identification apparatus 2 via the wireless channel W, for example. The communication part 18 functions as a transmission part that transmits the data indicating the intensity of the second infrared light to the glucose amount identification apparatus 2 that identifies the glucose amount in the measurement subject U's blood on the basis of the intensity of the second infrared light.

The storage 19 includes a Read Only Memory (ROM) and a Random Access Memory (RAM), for example. The storage 19 stores a program executed by the controller 20. The storage 19 may store a value of the duty used by the duty controller 202 to drive the current generator for infrared light 13 at the time when the measurement by the glucose amount identification apparatus 2 is finished.

The controller 20 is a Central Processing Unit (CPU), for example. The controller 20 functions as the data processor 201 and the duty controller 202 by executing the program stored in the storage 19.

The data processor 201 performs processing for notifying the duty controller 202 about the data inputted from the AD converter 17 and transmitting the data to the glucose amount identification apparatus 2 via the communication part 18. For example, the data processor 201 notifies the duty controller 202 about a value of the digital data indicating the intensity of the second infrared light, outputted from the infrared light receiver 15.

Further, the data processor 201 notifies the duty controller 202 about the value of the digital data corresponding to the current flowing through the infrared light emitter 11 and the red light emitter 12, outputted from the current generator for infrared light 13 and the current generator for red light 14. Furthermore, the data processor 201 causes the communication part 18 to transmit (i) the value of the digital data indicating the intensity of the second infrared light, outputted from the infrared light receiver 15 and (ii) the value of the digital data indicating the intensity of the second red light, outputted from the red light receiver 16.

The duty controller 202 generates the pulse voltage for causing the current generator for infrared light 13 to cause the pulse current to flow through the infrared light emitter 11. Further, the duty controller 202 generates the pulse voltage for causing the current generator for red light 14 to cause the pulse current to flow through the red light emitter 12. The duty controller 202 controls the duty of the pulse voltage applied to the current generator for infrared light 13 on the basis of the intensity of the second infrared light received by the infrared light receiver 15, to change the duty of the pulse current that the current generator for infrared light 13 causes to flow to the infrared light emitter 11. Specifically, the duty controller 202 applies, to the current generator for infrared light 13, the pulse voltage of the duty corresponding to the signal indicating the intensity of the second infrared light inputted from the infrared light receiver 15 via the AD converter 17 and the data processor 201.

The duty controller 202 controls the duty of the pulse voltage such that the intensity of the second infrared light is within an appropriate range. The duty controller 202 changes the duty of the pulse voltage from a first duty to a second duty on the basis of the intensity of the second infrared light that is (i) the light resulting from the first infrared light being reflected by the part of the measurement subject U's body or (ii) the light resulting from the first infrared light being transmitted through the part of the measurement subject U's body. Specifically, the duty controller 202 reduces the duty if the intensity of the second infrared light is excessively large, and increases the duty if the intensity of the second infrared light is excessively small. The duty controller 202 references a table stored in the storage 19, in which the intensity of the second infrared light is associated with the duty of the pulse voltage, to determine the duty corresponding to the intensity of the second infrared light received by the infrared light receiver 15, for example.

The duty controller 202 controls the current generator for red light 14 such that the current flows through the red light emitter 12 while the infrared light emitter 11 is not emitting the first infrared light and the current does not flow through the red light emitter 12 while the infrared light emitter 11 is emitting the first infrared light. By having the duty controller 202 operate in this manner, the infrared light emitter 11 and the red light emitter 12 do not emit light at the same time, which improves the accuracy of the intensity of the second infrared light detected while the infrared light emitter 11 is emitting the first infrared light.

Incidentally, the glucose amount identification apparatus 2 may measure the glucose amount in blood on the basis of the relationship between the intensity of the second infrared light and the intensity of the second red light in order not to erroneously detect the change of the second infrared light caused by a body condition (for example, body temperature or pulse) as the change of the glucose amount, for example. Therefore, the duty controller 202 changes the duty of the pulse voltage generated by the current generator for infrared light 13 on the basis of the difference between the intensity of the second infrared light and the intensity of the second red light such that the glucose amount identification apparatus 2 can measure the glucose amount on the basis of the relationship between the intensity of the second infrared light and the intensity of the second red light, for example.

Specifically, the duty controller 202 changes the duty of the pulse current that the current generator for infrared light 13 causes to flow to the infrared light emitter 11, such that the difference between the intensity of the second infrared light and the intensity of the second red light is equal to or greater than a threshold value corresponding to a difference sufficient to identify a change in the glucose amount. More specifically, the duty controller 202 increases the duty of the pulse voltage if the difference between the intensity of the second infrared light and the intensity of the second red light is less than the threshold value, and does not increase the duty of the pulse voltage if the difference is equal to or greater than the threshold value.

However, if the duty becomes excessively large, the temperature of the infrared light emitter 11 may become excessively high, and the wavelength of the first infrared light may exceed the appropriate range. Therefore, the duty controller 202 changes the duty of the pulse voltage such that an average current flowing through the infrared light emitter 11 is equal to or less than the maximum current value allowed for the infrared light emitter 11. The maximum current value allowed for the infrared light emitter 11 is the maximum value of the current within a range where the difference between the wavelength of the first infrared light radiated by the infrared light emitter 11 and a reference wavelength does not substantially affect the measurement of the glucose amount.

The duty controller 202 may determine the duty at the time when the pulse current starts to flow through the infrared light emitter 11, on the basis of the value of the duty at the time when the measurement by the glucose amount identification apparatus 2 is finished, which is stored in the storage 19. That is, the duty controller 202 may start generating the pulse voltage at the duty of the pulse current, stored in the storage 19, that was flowing in the infrared light emitter 11 when the second infrared light used for the previous measurement by the glucose amount identification apparatus 2 was acquired. By having the duty controller 202 start generating the pulse voltage at the duty stored in the storage 19 in this manner, the time required to set the duty to an optimum value is shortened, thus shortening the measurement time.

It should be noted that the duty controller 202 may stop generating the pulse voltage if an overcurrent of more than a prescribed value flows through the infrared light emitter 11 or the red light emitter 12. For example, the duty controller 202 prevents the current generator for infrared light 13 and the current generator for red light 14 from causing too much current to flow by stopping the generation of the pulse voltage if the signal indicating the magnitude of the current flowing through the infrared light emitter 11 generated by the current generator for infrared light 13 or the current flowing through the red light emitter 12 generated by the current generator for red light 14 indicates a current value equal to or higher than the prescribed value.

### [Operation of glucose amount identification apparatus 2]

The glucose amount identification apparatus 2 measures the glucose amount in the measurement subject U's blood on the basis of the intensity of the second infrared light. The glucose amount identification apparatus 2 identifies the intensity of the second infrared light by integrating the amount of the second infrared light received by the infrared light receiver 15 while the current generator for infrared light 13 is causing the pulse current to flow through the infrared light emitter 11. The glucose amount identification apparatus 2 identifies the glucose amount on the basis of, for example, the difference between (i) the intensity of the second infrared light identified on the basis of the integrated value of the amount of the second infrared light and (ii) the intensity of the second red light identified on the basis of the integrated value of the amount of the second red light received by the red light receiver 16 while the current generator for red light 14 is causing the pulse current to flow through the red light emitter 12.

The glucose amount identification apparatus 2 identifies the glucose amount on the basis of the relationship between the intensity of the second infrared light and the intensity of the second red light, for example. The glucose amount identification apparatus 2 outputs data indicating the measured glucose amount. The glucose amount identification apparatus 2 displays the glucose amount on a display or prints the glucose amount on a paper with a printer, for example.

### [Circuit examples of current generator for infrared light 13 and current generator for red light 14]

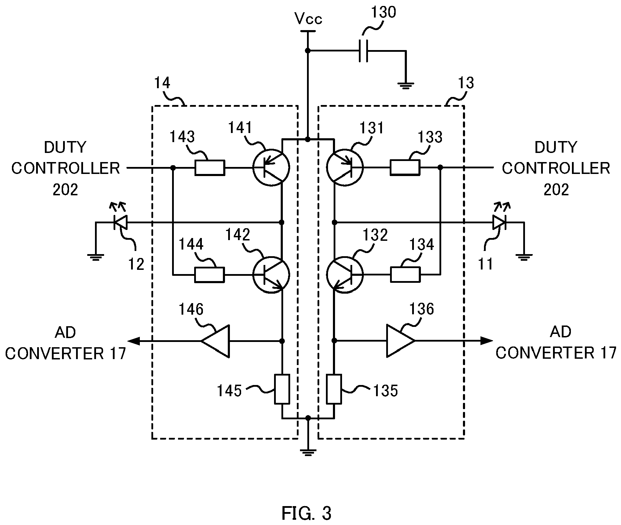

FIG. 3 shows circuit examples of the current generator for infrared light 13 and the current generator for red light 14. The current generator for infrared light 13 includes a transistor 131, a transistor 132, a resistor 133, a resistor 134, a resistor 135, and an amplifier 136. The current generator for red light 14 includes a transistor 141, a transistor 142, a resistor 143, a resistor 144, a resistor 145, and an amplifier 146.

FIG. 3 also shows a capacitor 130 provided between a power supply (Vcc) and a ground. The capacitor 130 is a large-capacity electrolytic capacitor that is charged with an electric charge to allow a large current to flow through the transistor 131, for example.

The circuits of the current generator for infrared light 13 and the current generator for red light 14 will be described in detail below. In the following description, the pulse voltage applied by the duty controller 202 to the current generator for infrared light 13 is referred to as a first pulse voltage, and the pulse voltage applied by the duty controller 202 to the current generator for red light 14 is referred to as a second pulse voltage.

The transistor 131 is a first infrared transistor that causes the current to flow through the infrared light emitter 11 while the first pulse voltage inputted from the duty controller 202 has a first polarity. In the example shown in FIG. 3, the transistor 131 is a PNP transistor, and is turned on to cause the current to flow through the infrared light emitter 11 while the first pulse voltage is 0 V. The transistor 131 is turned off while the first pulse voltage is at a high level voltage (for example, 3.3V which is the same as a power supply voltage), such that no current flows through the infrared light emitter 11. Since the resistance between a collector of the transistor 131 and a cathode of the infrared light emitter 11 is close to 0 while the transistor 131 is on, a large current based on the charge accumulated in the capacitor 130 can flow through the infrared light emitter 11.

The transistor 132 is a second infrared transistor that connects a terminal connected to the transistor 131 in the infrared light emitter 11 to the ground while the first pulse voltage has a second polarity different from the first polarity. In the example shown in FIG. 3, the transistor 132 is an NPN transistor, and is turned off while the first pulse voltage is 0 V.

The transistor 132 is turned on while the first pulse voltage is at the high level voltage, and connects an anode terminal of the infrared light emitter 11 to the ground through the amplifier 136. At the time when the transistor 132 is turned on, the transistor 131 is turned off, and the charge remaining on an anode side of the infrared light emitter 11 flows to the ground via the transistor 132 and the amplifier 136, thereby cutting off the current flowing through the infrared light emitter 11.

The resistor 133 and the resistor 134 function as base resistors. A speed-up capacitor may be connected in parallel to the resistor 133 and the resistor 134.

The resistor 135 is a resistor for limiting the current. The resistor 135 has a resistance value smaller than the impedance of the infrared light emitter 11 in a frequency range corresponding to a pulse width of the first pulse voltage such that the charge remaining in the infrared light emitter 11 flows to the ground while the transistor 132 is turned on.

The amplifier 136 generates the voltage corresponding to the current flowing through the transistor 132 while the transistor 132 is on. The amplifier 136 inputs the generated voltage to the AD converter 17. Since the current flowing through the transistor 132 correlates with the magnitude of the current flowing through the infrared light emitter 11 while the transistor 131 is on, the voltage generated by the amplifier 136 is used by the duty controller 202 to detect that an overcurrent flows through the infrared light emitter 11.

The transistor 141 is a first red transistor that causes the current to flow through the red light emitter 12 while the second pulse voltage inputted from the duty controller 202 has the first polarity. In the example shown in FIG. 3, the transistor 141 is a PNP transistor, and is turned on to cause the current to flow through the red light emitter 12 while the second pulse voltage is 0 V. The transistor 141 is turned off while the second pulse voltage is at the high level voltage, such that no current flows through the red light emitter 12. Since the resistance between the collector of the transistor 141 and the cathode of the red light emitter 12 is close to 0 while the transistor 141 is on, a large current can be caused to flow through the red light emitter 12.

The transistor 142 is the second infrared transistor that connects a terminal connected to the transistor 141 in the red light emitter 12 to the ground while the second pulse voltage has the second polarity. In the example shown in FIG. 3, the transistor 142 is an NPN transistor, and is turned off while the second pulse voltage is 0 V.

The transistor 142 is turned on while the second pulse voltage is at the high level voltage, and connects the anode terminal of the red light emitter 12 to the ground via the amplifier 146. At the time when the transistor 142 is turned on, the transistor 141 is turned off, and the charge remaining on the anode side of the red light emitter 12 flows to the ground via the transistor 142 and the amplifier 146, thereby cutting off the current flowing through the red light emitter 12.

The resistor 143 and the resistor 144 function as the base resistors. The speed-up capacitor may be connected in parallel to the resistor 143 and the resistor 144.

The resistor 145 is a resistor for limiting the current. The resistor 145 has a resistance value smaller than the impedance of the red light emitter 12 in the frequency range corresponding to the pulse width of the second pulse voltage, such that the charge remaining in the red light emitter 12 flows to the ground while the transistor 142 is turned on.

The amplifier 146 generates the voltage corresponding to the current flowing through the transistor 142 while the transistor 142 is on. The amplifier 146 inputs the generated voltage to the AD converter 17. Since the current flowing through the transistor 142 correlates with the magnitude of the current flowing through the red light emitter 12 while the transistor 141 is on, the voltage generated by the amplifier 146 is used for the duty controller 202 to detect that an overcurrent flows through the red light emitter 12.

### [Processing of controller 20]

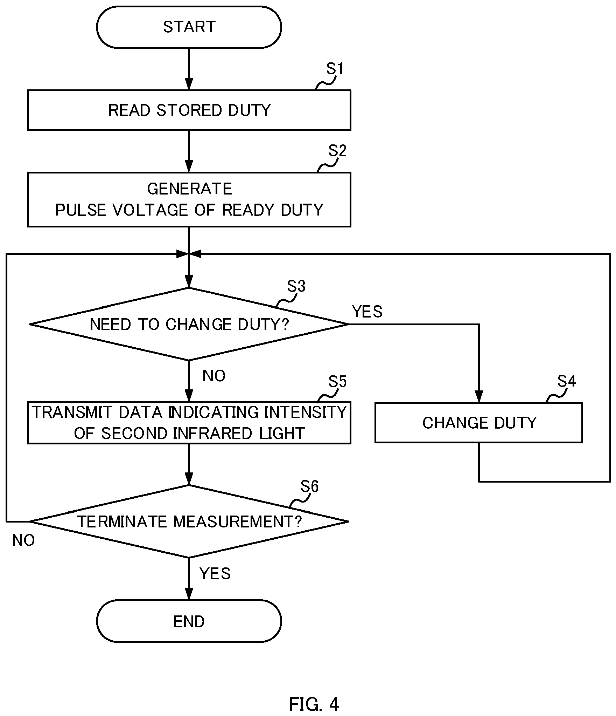

FIG. 4 is a flowchart that shows processing executed by the controller 20. For example, the flowchart shown in FIG. 4 starts when the measurement subject U performs an operation for starting the measurement of the glucose amount in the glucose amount identification apparatus 2, and the data processor 201 receives an instruction to start the measurement from the glucose amount identification apparatus 2.

The duty controller 202 reads the duty (first duty) stored in the storage 19 (step S1), and starts generating the pulse voltage of the read duty (step S2). Subsequently, the duty controller 202 determines whether or not it is necessary to change the duty on the basis of the intensity of the second infrared light inputted from the infrared light receiver 15 (step S3).

If the duty controller 202 determines in step S3 that the duty needs to be changed (YES in step S3), the duty controller 202 changes the duty such that the intensity of the second infrared light becomes close to an appropriate value (step S4), and again executes the process in step S3. If the duty controller 202 determined in step S3 that it is not necessary to change the duty (NO in step S3), the duty controller 202 causes the data processor 201 to transmit the data indicating the intensity of the second infrared light inputted from the infrared light receiver 15, to the glucose amount identification apparatus 2 (step S5).

The data processor 201 and the duty controller 202 repeat the processing from step S3 to step S5 until the data processor 201 receives an instruction to terminate the measurement from the glucose amount identification apparatus 2 (during NO in step S6). When the data processor 201 receives the instruction to terminate the measurement (YES in step S6), the data processor 201 and the duty controller 202 terminate the operation.

### [First variation]

In the above description, an example is shown in which the measurement system S includes the measurement apparatus 1 and the glucose amount identification apparatus 2, but the measurement system S may instead be formed by only the measurement apparatus 1. In this case, the controller 20 of the measurement apparatus 1 operates as the glucose amount identification apparatus 2.

### [Second variation]

In the above description, the measurement apparatus 1 includes the red light emitter 12, the current generator for red light 14, and the red light receiver 16, but the measurement apparatus 1 does not have to include the red light emitter 12, the current generator for red light 14, and the red light receiver 16. That is, the measurement system S may measure the glucose amount using only the infrared light.

### [Third variation]

In the above description, the current generator for infrared light 13 and the current generator for red light 14 include a bipolar transistor, but the current generator for infrared light 13 and the current generator for red light 14 may be switched between the on state and the off state by a semiconductor switch other than the bipolar transistor.

### [Fourth variation]

The measurement apparatus 1 is attached to the measurement subject U's wrist in the above description, but the part to which the measurement apparatus 1 is attached is not limited to the wrist, and may be any other part as long as the infrared light can be radiated to the blood.

### [Fifth variation]

The case where the controller 20 includes a CPU is shown as an example in the above description, but the controller does not have to include the CPU and may be composed of electronic elements other than the CPU.

### [Effect of measurement apparatus 1]

As described above, the measurement apparatus 1 includes the current generator for infrared light 13 capable of causing the large current to flow in a pulse shape through the infrared light emitter 11, and the duty controller 202 controls the duty of the pulse current generated by the current generator for infrared light 13 on the basis of the intensity of the infrared light detected by the infrared light receiver 15. By having the duty controller 202 operate in this manner, the large current flows through the infrared light emitter 11 to increase the light emission intensity of the infrared light emitter 11, while the temperature of the infrared light emitter 11 is prevented from increasing excessively. As a result, the wavelength of the pulse light or the wavelength of the light absorbed by the glucose becomes a wavelength suitable for detecting the glucose, such that the measurement accuracy of the glucose amount is improved.

The present invention is explained on the basis of the exemplary embodiments. The technical scope of the present invention is not limited to the scope explained in the above embodiments and it is possible to make various changes and modifications within the scope of the invention. For example, all or part of the apparatus can be configured with any unit which is functionally or physically dispersed or integrated. Further, new exemplary embodiments generated by arbitrary combinations of them are included in the exemplary embodiments of the present invention. Further, effects of the new exemplary embodiments brought by the combinations also have the effects of the original exemplary embodiments.

### [Description of Symbols]

1 measurement apparatus
2 glucose amount identification apparatus
11 infrared light emitter
12 red light emitter
13 current generator for infrared light
14 current generator for red light
15 infrared light receiver
16 red light receiver
17 AD converter
18 communication part
19 storage
20 controller
130 capacitor
131 transistor
132 transistor
133 resistance
134 resistance
135 resistance
136 amplifier
141 transistor
142 transistor
143 resistance
144 resistance
145 resistance
146 amplifier
201 data processor
202 duty controller

## Claims

1. A measurement system comprising:
an infrared light emitter that emits first infrared light;
an infrared light receiver that receives second infrared light that is (i) light resulting from the first infrared light being reflected by a part of a body of a measurement subject or (ii) light resulting from the first infrared light being transmitted through a part of the body of the measurement subject;
a current generator for infrared light that causes a pulse current to flow to the infrared light emitter;
a duty controller that changes a duty of the pulse current caused to flow by the current generator for infrared light on the basis of an intensity of the second infrared light; and
a glucose amount identification part that identifies a glucose amount in blood of the measurement subject on the basis of an intensity of the second infrared light.

2. The measurement system according to claim 1, further comprising:
a red light emitter that emits first red light;
a red light receiver that receives second red light that is (i) light resulting from the first red light being reflected by a part of the body of the measurement subject or (ii) light resulting from the first red light being transmitted through a part of the body of the measurement subject; and
a current generator for red light that causes a pulse current to flow through the red light emitter, wherein
the duty controller changes the duty of the pulse current caused to flow through the infrared light emitter by the current generator for infrared light such that the glucose amount identification part can measure the glucose amount on the basis of the relationship between the intensity of the second infrared light and the intensity of the second red light.

3. The measurement system according to claim 2, wherein
the duty controller controls the current generator for red light such that the current generator for red light (i) causes a current to flow to the red light emitter to cause the red light emitter to radiate the first red light while the infrared light emitter is not emitting the first infrared light and (ii) causes a current not to flow to the red light emitter while the infrared light emitter is emitting the first infrared light.

4. The measurement system according to any one of claims 1 to 3, wherein
the duty controller changes the duty of the pulse current such that an average current flowing through the infrared light emitter is equal to or less than the maximum current value allowed for the infrared light emitter.

5. The measurement system according to any one of claims 1 to 4, wherein
the duty controller generates a pulse voltage of a duty corresponding to the duty of the pulse current,
the current generator for infrared light includes:
a first infrared transistor that causes a current to flow to the infrared light emitter while the pulse voltage has a first polarity, and
a second infrared transistor that connects a terminal connected to the first infrared transistor in the infrared light emitter to a ground while the pulse voltage has a second polarity different from the first polarity.

6. The measurement system according to any one of claims 1 to 5, wherein
the glucose amount identification part identifies the intensity of the second infrared light by integrating an amount of the second infrared light received by the infrared light receiver while the current generator for infrared light is causing a pulse current to flow through the infrared light emitter.

7. The measurement system according to any one of claims 1 to 6, further comprising:
a storage that stores a value of the duty at a time when measurement by the glucose amount identification part is finished, wherein
the duty controller determines the duty at a time when the pulse current starts to flow through the infrared light emitter, on the basis of the value of the duty stored in the storage.

8. A measurement apparatus comprising:
an infrared light emitter that emits first infrared light;
an infrared light receiver that receives second infrared light that is (i) light resulting from the first infrared light being reflected by a part of a body of a measurement subject or (ii) light resulting from the first infrared light being transmitted through a part of the body of the measurement subject;
a current generator for infrared light that causes a pulse current to flow through the infrared light emitter;
a duty controller that changes a duty of the pulse current caused to flow by the current generator for infrared light on the basis of an intensity of the second infrared light; and
a transmission part that transmits data indicating the intensity of the second infrared light to a glucose amount identification apparatus that identifies a glucose amount in blood of the measurement subject on the basis of the intensity of the second infrared light.

9. A program for causing a computer to execute the steps of:
generating a pulse voltage of a first duty to be inputted to a current generator for infrared light that drives an infrared light emitter that emits first infrared light;
changing a duty of the pulse voltage from the first duty to a second duty on the basis of an intensity of second infrared light that is (i) light resulting from the first infrared light being reflected by a part of a body of a measurement subject or (ii) light resulting from the first infrared light being transmitted through a part of the body of the measurement subject; and
transmitting data indicating the intensity of the second infrared light to a glucose amount identification apparatus for identifying a glucose amount in blood of the measurement subject on the basis of the intensity of the second infrared light.
